(19) Europäisches Patentamt
European Patent Office
Office européen des brevets

(11) Publication number: 0 219 146
B1

# (12) EUROPEAN PATENT SPECIFICATION

(45) Date of publication of the patent specification: 31.10.90

(51) Int. Cl.[5]: C11B 9/00, A23L 1/226, A24B 15/40, A61K 7/46, C07D 333/08

(21) Application number: 86201449.5

(22) Date of filing: 21.08.86

## (54) Substituted thiophenes, flavouring and perfuming compositions, perfumed products containing them.

(30) Priority: 16.09.85 NL 8502530

(43) Date of publication of application: 22.04.87 Bulletin 87/17

(45) Publication of the grant of the patent: 31.10.90 Bulletin 90/44

(84) Designated Contracting States: CH DE FR GB LI NL SE

(56) References cited:
FR-A- 2 012 074
GB-A- 1 156 481

CHEMISTRY AND INDUSTRY, no. 16, 18th August 1979, pages 552,553, Society of Chemical Industry, London, GB; T.L. PEPPARD: "Facile formation of sulphur-containing heterocycles from myrcene and their occurrence in the essential oil of hops"
JOURNAL OF THE CHEMICAL SOCIETY, PERKIN TRANSACTIONS I, 1982, pages 1089-1094; J.A. ELVIDGE et al.: "Chemistry of hop constituents. Part 43.1 Cyclic polysulphides and a thiophen from myrcene, and their occurrence in the essential oil of hops"
JOURNAL OF AGRICULTURAL AND FOOD CHEMISTRY, vol. 28, 1980, pages 237-242, American Chemical Society; T. SHIBAMOTO: "Heterocyclic

(73) Proprietor: NAARDEN INTERNATIONAL N.V., P.O.Box 2 Huizerstraatweg 28, NL-1400 CA Naarden-Bussum(NL)

(72) Inventor: Van den Heuvel, Henry Lodewijk Alexander, Arminiushof 49, NL-1216 KD Hilversum(NL)
Inventor: Jaegers, Paulus Paulines Jozef Maria, Varenstraat 33, NL-3765 WJ Soest(NL)

(74) Representative: Dries, Antonius Johannes Maria, Unilever N.V. Patent Division P.O. Box 137, NL-3130 AC Vlaardingen(NL)

(56) References cited: (continuation)
compounds found in cooked meats"
RECUEIL DES TRAVAUX CHIMIQUES DES PAYS-BAS, vol. 103, 7th-8th July/August 1984, pages 220-224; C.G.M. JANSSEN et al.: "SP3-carbon detosylation by dibal-H: model studies"

EP 0 219 146 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to flavouring and perfume compositions, not being hop oil, which contain one or more 3-alkyl-, 3-alkenyl - and 3-alkadienyl-substituted thiophenes and to foodstuffs and semiluxuries flavoured with one or more of these compounds or with said compositions and to products perfumed therewith. In addition, the invention relates to some 3-alkyl-, 3-alkenyl- and 3-alkadienyl-substituted thiophenes.

There is an on-going interest in the preparation of synthetic flavours and fragrances and the application thereof in foodstuffs and semiluxuries and in products to be perfumed such as cosmetics, soaps, detergents, household products and the like. This interest is stimulated by the insufficient quantity and often varying quality of natural flavours and fragrances. Although sulphur compounds per se generally do not have a pleasant taste and odour, it has nevertheless emerged that in many natural flavours and odours they play an essential role. It also appears that in many perfumes a somewhat "sulphurous" note is desirable to complete the total odour. There is therefore a requirement for compounds with a clearly pleasant sulphurous odour and flavour note which, however, does not come too strongly to the fore, preferably combined with other, pleasant olfactory properties so that the compounds can easily be combined with known fragrances and flavours.

Surprisingly, it has now been found that 3-substituted thiophenes with the general formula I

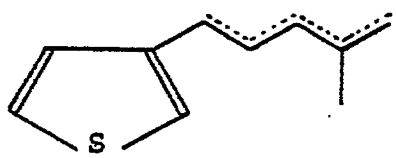

(I)

have organoleptically very valuable properties. as a result of which they are very suitable for use as fragrances or flavours.

In formula I none, one or two of the dotted lines represents a double bond and in particular in a manner such that no cumulated double bonds are present. The compounds represented in this manner may have both the E and the Z configuration.

Such substituted thiophenes are hardly known in the perfume and flavour industry. U.S. Patent 3,702,253 makes mention of 3-vinylthiophene which can be used as a flavour in foodstuffs and drinks and has a hydrocarbon-like odour or taste.

Said compound, however, has chemical structure quite a different the 3-(4-methylpentyl)thiophene, 3-(4-methylpentenyl)thiophene and 3-(4-methyl-pentadienyl)thiophene of the present invention.

3-(4-Methylpent-3-enyl)thiophene has been detected by various investigators in hop oil (see A. Suggett, M. Moir, J.C. Seaton, Proceedings of the European Brewery Convention Congress, Berlin, 1979, pages 79-89; T.L. Peppard, J.A. Elvidge, Chem. & Ind. 1979, pages 552-3; S. Araki, Y. Butsugan, Bull. Chem. Soc. Japan, 56 (1983), pages 1446-9 and J.A. Elvidge, S.P. Jones, J. Chem. Soc. Perkin I, 1982, pages 1089-94). In the last publication the compound is described as a "pungent oil". Suggett and his coworkers, who first detected this and some other sulphur compounds in hop, report that they give an undesirable flavour when added to beer.

The other compounds according to the invention were hitherto unknown.

It was not possible to suppose therefore that the compounds according to the invention would have such valuable and varied organoleptic properties which, in addition to a general, but not very pronounced sulphurous note contain, inter alia, flowery, green, spicy, citrus-like, malty and caramel-like odour and taste notes. Thus, the abovementioned 3-(4-methylpent-3-enyl)thiophene is characterized by a pleasant green and citrus-like odour and a taste which clearly has citrus aspects and the organolepsis of Z-3-(4-methylpenta-1,3-dienyl)thiophene exhibits a green and fresh-flowery character, but that of E-3-(4-methylpenta-1,3-dienyl)thiophene exhibits a spicy, malty, caramel-like character.

The compounds according to the invention can be used as such as flavours and fragrances, or they can first be mixed with suitable carriers or diluents. They can also be combined with other single compounds or with mixtures, for example with essential oils, in a usual manner for the formation of flavouring or perfume compositions. In this connection the terms "flavouring composition" and "perfume compositions" mean mixtures of flavours or fragrances respectively and/or essential oils, if desired dissolved in a suitable solvent or mixed with a powdered substrate or processed to form a powdered product and used to impart a desired taste or odour to products of all types, or to reinforce or to improve the taste or odour which these products already have. Products to be flavoured are foodstuffs and semiluxuries, by which in this connection are meant: solid or liquid products intended for human consumption, including tobacco products, medicines and toothpaste. Products to be perfumed are, for example, soaps, detergents, air-fresheners, room sprays, pomanders, candles, cosmetics such as creams, ointments, toilet waters, pre- and after-shave lotions, talcum powders, hair-care agents, body deodorants and antiperspirants.

Basic perfume and flavouring substances which can be advantageously combined with the compounds according to the invention are, for example, natural products such as extracts, essential oils, absolutes resinoids, resins, concretes etc., but also synthetic basic substances such as hydrocarbons, alcohols, aldehydes, ketones, ethers, acids, esters, acetals, ketals, nitriles, etc., including saturated and unsaturated compounds, aliphatic, carbocyclic and heterocyclic compounds.

Such basic materials are mentioned, for example, in S. Arctander, Perfume and Flavor Chemicals (Montclair, N.J., 1969), in S. Arctander, Perfume and Flavor Materials of Natural Origin (Elizabeth, N.J., 1960) and in T.E. Furia et al., CRC Fenaroli'S Handbook of Flavor Ingredients, 2nd edition (Cleveland, CRC Press Inc., 1975).

Examples of fragrances which can be used in combination with the compounds according to the invention are geraniol, geranyl acetate, linalol, linalyl acetate, tetrahydrolinalol, citronellol, citronellyl acetate, dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, styrallyl acetate, benzyl benzoate, amyl salicylate, dimethylbenzyl-carbinol, trichloromethylphenylcarbinyl acetate, p-tert-butyl cyclohexyl acetate, isononyl acetate, vetiveryl acetate, vetiverol, α-hexylcinnamaldehyde, 2-methyl-3-(p-tert-butylphenyl)propanal, 2-methyl-3-(p-isopropylphenyl)propanal, 3-(p-tert-butylphenyl)propanal, tricyclodecenyl acetate, tricyclodecenyl propionate, 4-(4-hydroxy-4-methylpentyl)-3-cyclohexenecarbaldehyde, 4-(4-methyl-3-pentenyl)-3-cyclohexenecarbaldehyde, 4-acetoxy-3-pentyltetrahydropyran, 3-carboxymethyl-2-pentylcyclopentane, 2-n-heptylcyclopentanone, 3-methyl-2-pentyl-2-cyclopentenone, n-decanal, n-dodecanal, 9-decenol-1, phenoxyethyl isobutyrate, phenylacetaldehyde dimethylacetal, phenylacetaldehyde diethylacetal, geranyl nitrile, citronellyl nitrile, cedryl acetate, 3-isocamphylcyclohexanol, cedryl methyl ether, isolongifolanone, aubepine nitrile, aubepine, heliotropin, coumarin, eugenol, vanillin, diphenyl oxide, hydroxycitronellal, ionones, methylionones, isomethylionones, irones, cis-3-hexenol and esters thereof, indan musks tetralin musks isochroman musks macrocyclic ketones, macrolactone musks ethylene brassylate, aromatic nitromusks.

Auxiliary materials and solvents which can be used in perfume compositions which contain compounds according to the invention are, for example: ethanol, isopropanol, diethyleneglycol monoethyl ether, diethyl phthalate etc. Such auxiliary materials and solvents for aroma compositions are, for example: ethanol, diethyleneglycol monoethyl ether, propylene glycol, glycerol and triacetin.

The quantities of the compounds according to the invention to be used may be strongly divergent and depend, inter alia, on the product in which the compounds are used and on the nature and the quantity of the other components of a flavouring or perfume composition.

In most cases a quantity of 0.05 parts by weight per million of a compound according to the invention in foodstuffs and semiluxuries will already be perceptible. In concentrated flavouring compositions a quantity of up to 5 % by weight can be used with advantage in some cases.

In concentrated perfume compositions quantities of 100 parts by weight per million may already have a perceptible effect on the odour impression. On the other hand, to achieve special odour effects it is also possible to use a quantity of 5 % by weight or even more in a composition. In products perfumed with the aid of perfume compositions these concentrations are proportionally lower depending on the quantity used of the composition in the finished product.

The compounds according to the invention can be prepared according to methods usual for such compounds, for example by the Wittig reaction of 3-thienylcarbaldehyde or 3-thienylacetaldehyde with an ylide prepared from a suitably chosen alk(en)yltriphenylphosphonium halide. Insofar as it is possible for the compounds according to the invention to be (Z)- and (E)-isomers, the above-mentioned synthesis methods yield a mixture of said isomers. The ratio of isomers in the mixture can be influenced by changes in the reaction procedure. Thus, more of the (Z)-isomer is obtained if the reaction is carried out in a nonpolar solvent and, on the other hand, more of the (E)-isomer is obtained in polar solvents and/or in the presence of lithium salts, for example by making use of a lithium base for the preparation of the ylide. The Wittig reaction is described in J. March, Advanced Organic Chemistry, 2nd edition, pages 864-872, in particular page 870, and in the literature mentioned therein.

The 4-methylpentyl and some 4-methylpentenyl compounds may be prepared by a Wurtz-type reaction between 3-halomethylthiophene, a 3-methylbut(en)yl halide and magnesium.

3-(4-Methylpent-3-enyl)thiophene can be prepared according to the instructions of S. Araki and Y. Butsugan (see above) or of A. Corvers, J.H. van Mil, M.M.E. Sap and H.M. Buck, Rec. Trav. Chim. Pays Bas, 96 (1977), pages 18-22.

Isomeric mixtures obtained in the preparation of compounds according to the invention may be separated into the separate isomers in a conventional manner, for example by means of preparative GLC. As a rule, however, such a separation is not necessary and the isomeric mixture can be used with advantage as a flavouring or perfume.

The following examples are exclusively intended to illustrate the invention and the synthesis of the compounds according to the invention.

Example I

Preparation of 3-(4-methylpent-3-enyl)thiophene

550 g of cyclohexane, 231 g of 3-methylthiophene and 5 g of azoisobutyronitrile were introduced into a 2-litre reaction flask. The mixture was heated while stirring thoroughly until it refluxed, after which a mixture of 437 g of N-bromosuccinamide and 5 g of azoisobutyronitrile was added at a rate such that the reaction mixture continued to reflux.

After everything had been added, the reaction mixture was cooled to 10°C, then the precipitated succinamide was filtered off and washed with cyclohexane. The combined filtrate was evaporated down under reduced pressure and the evaporation residue was distilled from 2 g of calcium carbonate. 217 g (52%) of 3-bromomethylthiophene were obtained; b.p. 42-48°C/0.2 kPa.

217 g of 3-bromomethylthiophene, 513 g of prenyl chloride, 117 g of copper(I) iodide and 2 kg of tetrahydrofuran were introduced into a 4-litre reaction flask. 750 g of magnesium shavings were then added in small portions at a rate such that the temperature of the reaction mixture did not rise above 30°C. If the temperature rises were too great, the reaction mixture was temporarily cooled. The first portion of magnesium was activated by subliming an iodine crystal onto it. After all the magnesium had been added, a check was made by means of GLC as to whether bromomethylthiophene or prenyl chloride was still present. It was possibly necessary to add more magnesium. The mixture was then stirred for a further hour at 40°C and after that poured out into a mixture of 180 g of ammonium chloride, 1 litre of water and 500 g of ice, and stirred.

The aqueous layer was separated and extracted with 800 g of cyclohexane. The combined organic layers were evaporated down under reduced pressure. A fraction of 142 g boiling at 48-60°C/0.15 kPa, was collected. This was fractionated under reduced pressure, 112 g of 3-(4-methylpent-3-enyl)thiophene being collected; b.p. 68°C/0.3 kPa (yield 55%).

Example II

Preparation of 3-(4-methylpenta-1,3-dienyl)thiophene

Prenyltriphenylphosphonium chloride was prepared according to the instructions of R. Rüegg, U. Schweiter, G. Rijser, P. Schudel and 0. Isler; Helv. Chim. Acta., 44, 994 (1961).

73.4 g of prenyltriphenylphosphonium chloride, 7.8 g of sodium amide and 260 ml of dry tetrahydrofuran were introduced into a 1-litre reaction flask. The reaction mixture was kept under a dry nitrogen atmosphere, stirred at room temperature for 30 minutes and then cooled to approx. -15°C. A solution of 19.8 g of 3-thienylcarbaldehyde in 50 ml of dry tetrahydrofuran was then added in measured quantities in a manner such that the temperature of the reaction mixture remained below -10°C. After this addition, the reaction mixture was stirred for a further 4 hours at room temperature. 200 ml of water were then added, after which the organic layer was separated. The aqueous layer was extracted 3 times with ether. The combined organic layers were dried over magnesium sulphate and then evaporated down under reduced pressure. From the evaporation residue the precipitated triphenylphosphine oxide was removed by boiling up five times with petroleum ether and filtering. The filtrate was evaporated down, 22 g of evaporation residue being obtained which was distilled under reduced pressure. 12 g (42%) of 3-(4-methylpenta-1,3-dienyl)thiophene, b.p. 96-97°C/0.15 kPa were obtained consisting of a mixture of 60% (Z)-isomer and 40% (E)-isomer. The mixture exhibited a spicy and fresh-flowery character and was as such very suitable for use as a flavour or fragrance.

It was possible to separate it into the two pure components by means of GLC through a packed column containing 10% DEGS as the stationary phase (2 m, column temperature 165°C, gas flow rate 60 ml/min), the (Z)-isomer being obtained first.

(E)-isomer: m.p. 31-32°C

NMR ($CCl_4$, 100 MHz, $\delta$ in ppm with respect to TMS): 1.81 (6H, broad s); 5.82 (1H, broad d, J = 10 Hz); 6.30 (1H, d, J = 15 Hz); 6.67 (1H, dd, J = 10 and 15 Hz); 6.97 (1H); 7.13 (2H).

(Z)-isomer: n 20/D = 1.6075.

NMR: 1.79 (3H, broad s); 1.81 (3H, broad s); 6.0-6.4 (3H, m); 6.9-7.2 (3H, m).

Example III

A grapefruit flavouring for a soft drink was prepared according to the recipe below:

1.7 g of grapefruit juice oil
4.0 g of lime extract
17.0 g of orange extract
110.0 g of grapefruit oil extract
390.0 g of ethanol

The above components were mixed and stirred until quite homogeneous. The mixture was then made up to 1 kg with demineralized water while still stirring.

From the abovementioned flavouring a grapefruit drink was prepared by adding 2 g of the aroma to 1 litre of water containing carbonic acid.

A grapefruit drink was prepared in the same manner starting from a grapefruit flavouring that in addition to the abovementioned components contained also 0.3 g of 3-(4-methylpent-3-enyl)thiophene per kg of flavouring.

Both grapefruit drinks were compared by a panel of experts. It was generally agreed that the grapefruit drink which contained 3-(4-methylpent-3-enyl)thiophene has a more powerful and more complete grapefruit taste.

Example IV

A perfume composition for a man's cologne was prepared according to the recipe below:

| | | | |
|---|---|---|---|
| Acetyl cedrene | 150 | parts | by weight |
| Bergamot oil, furocoumarin-free | 100 | " | " |
| Linalyl acetate | 50 | " | " |
| Lavandin oil | 50 | " | " |
| Geranium oil, bourbon | 50 | " | " |
| Lemon oil | 50 | " | " |
| Coumarin | 30 | " | " |
| Lavendar oil, French | 30 | " | " |
| 2-Methyl-3-(p-tert-butylphenyl)-propanal | 25 | " | " |
| Heliotropin | 20 | " | " |
| Dihydromyrcenol | 20 | " | " |
| 11-Oxahexadecanolide | 20 | " | " |
| Lime oil | 20 | " | " |
| Vetiveryl acetate | 20 | " | " |
| Petitgrain oil | 20 | " | " |
| Methyl 2,4-dihydroxy-3,6-dimethylbenzoate | 10 | " | " |
| Coriander oil | 10 | " | " |
| Sandelwood oil, East Indian | 10 | " | " |
| Eugenol | 10 | " | " |
| Sagebrush oil | 10 | " | " |
| Methylnonylacetaldehyde* | 10 | " | " |
| Methylchavicol | 5 | parts | by weight |
| 3-(4-Methylpent-3-enyl)thiophene* | 20 | " | " |
| | 750 | parts | by weight |

* 10% solution in dipropylene glycol

## Claims

1. Flavouring and perfume compositions, not being hop oil, characterized by a content of one or more thiophene derivatives having the formula:

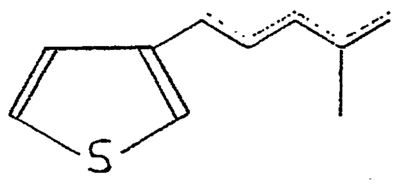

wherein none, one or two of the dotted lines represent a double bond, with the proviso that no cumulated double bonds are present.

2. Perfume compositions according to claim 1, characterized by a content of at least 100 ppm of one or more of said thiophene derivatives.

3. Flavoured foodstuffs and semiluxuries, characterized by a content of at least 0.05 ppm of one or more thiophene derivatives having the formula:

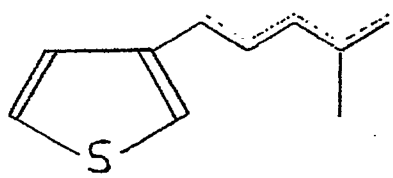

wherein none, one or two of the dotted lines represent a double bond, with the proviso that no cumulated double bonds are present.

4. Use of a flavouring composition according to claim 1 for flavouring foodstuffs and semiluxuries.

5. Use of a perfume composition according to claims 1 or 2 for preparing perfumed products.

6. 3-(4-methylpenta-1,3-dienyl)thiophene.

## Patentansprüche

1. Geschmacks- und Duftzusammensetzungen, die nicht Hopfenöl sind, gekennzeichnet durch einen Gehalt an einem oder mehreren Thiophenderivaten der Formel

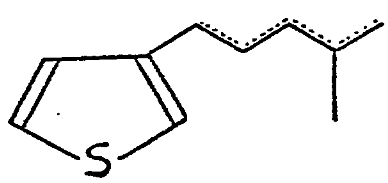
,

worin keine, eine oder zwei der punktierten Linien eine Doppelbindung mit der Maßgabe bedeuten, daß keine kumulierten Doppelbindungen vorliegen.

2. Duftzusammensetzung nach Anspruch 1, gekennzeichnet durch einen Gehalt von mindestens 100 ppm eines oder mehrerer dieser Thiophenderivate.

3. Aromatisierte Nahrungsmittel und Artikel des gehobenen Verbrauches, gekennzeichnet durch einen Gehalt von mindestens 0,05 ppm eines oder mehrerer Thiophenderivate der Formel

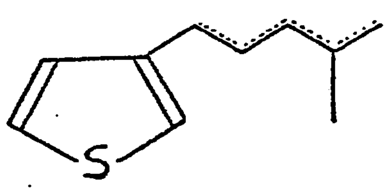
,

worin keine, eine oder zwei der punktierten Linien eine Doppelbindung mit der Maßgabe bedeuten, daß keine kumulierten Doppelbindungen vorliegen.

4. Verwendung einer Geschmackszusammensetzung nach Anspruch 1 zum Aromatisieren von Nahrungsmitteln und Artikeln des gehobenen Verbrauches.

5. Verwendung einer Duftzusammensetzung nach Anspruch 1 oder 2 zur Herstellung parfümierter Produkte.
6. 3-(4-Methylpenta-1,3-dienyl)thiophen.

**Revendications**

1. Compositions aromatisantes et parfumantes, qui ne sont pas une essence de houblon, caractérisées par une teneur en un ou plusieurs dérivé(s) de thiophène répondant à la formule:

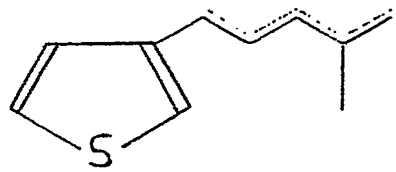

dans laquelle aucune des lignes en pointillés ou une ou deux de ces lignes représentent une double liaison, à la condition de l'absence de doubles liaisons cumulées.
2. Compositions parfumantes selon la revendication 1, caractérisée par une teneur d'au moins 100 ppm en un ou plusieurs desdits dérivés du thiophène.
3. Aliments et produits de semi-luxe aromatisés caractérisés par une teneur d'au moins 0,05 ppm en un ou plusieurs dérivés de thiophène de formule:

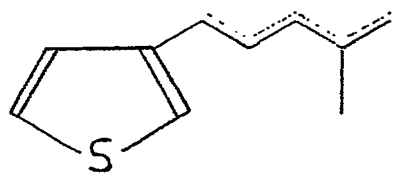

dans laquelle aucune des lignes en pointillés ou une ou deux de ces lignes représentent une double liaison, à la condition de l'absence de doubles liaisons cumulées.
4. Utilisation d'une composition aromatisante selon la revendication 1, pour aromatiser des aliments et des produits de semi-luxe.
5. Utilisation d'une composition parfumante selon la revendication 1 ou 2, pour préparer des produits parfumés.
6. 3-(4-méthylpenta-1,3-diényl)thiophène.